# EUROPEAN PATENT APPLICATION

(11) **EP 2 426 979 A1**
(43) Date of publication of application: **07.03.2012**
(21) Application number: 10769542.1
(22) Date of filing: 15.02.2010
(51) Int. Cl.: H04W 24/04, H04W 4/04, H04W 84/10

(54) **WIRELESS COMMUNICATION DEVICE AND WIRELESS COMMUNICATION METHOD**

(30) Priority: 30.04.2009 JP 2009110913
(71) Applicant: Fujitsu Limited, Kawasaki-shi, Kanagawa 211-8588 (JP)
(72) Inventor: IDA, Ichirou, Kawasaki-shi Kanagawa 211-8588 (JP); YOKOO, Kaoru, Kawasaki-shi Kanagawa 211-8588 (JP)
(74) Representative: Ward, James Norman
(86) International application number: PCT/JP2010/052229
(87) International publication number: WO 2010/125842

(57) **Abstract**

Provided is a technology of accurately grasping a transmission status when shadowing occurs. A wireless communication device includes transmitting means wirelessly transmitting test signals a predetermined number of times at an interval shorter than a transmission interval of data signals and measuring means measuring reception status of a plurality of acknowledgement signals transmitted back in response to the respective test signals transmitted by the transmitting means.

## Description

### [Technical Field]

The embodiments relate to a wireless communication technology.

### [Background Art]

A near-distance wireless communication network configured by a sensor device which acquires biometric information and a gateway device which collects pieces of biometric information from the sensor device and transmits the biometric information to another network, is called a body area network (BAN). With utilization of this BAN, for example, it is feasible to realize an application which schemes to improve efficiency of an inspection at a medical institution or to enrich health management outside the medical institution by measuring the biometric information such as heart beats and a body temperature at all times.

As communication methods used for the BAN system, there exists a method of utilizing electromagnetic waves propagating in the air spaced away from the living body, a method of using the electromagnetic waves propagating along the surface of the living body, and so on.

In the wireless communication system such as the BAN system using a spatial transmission path, it is of importance to accurately grasp a transmission status in order to shorten a period of communication-disabled time. Such being the case, some methods are disclosed for grasping the transmission status (refer to Patent documents 1 through 4 which follow)

### [Documents of Prior Arts]

### [Patent document]

[Patent document 1] Japanese Laid-Open Patent Publication No.2002-281047
[Patent document 2] Japanese Laid-Open Patent Publication No.2005-72658
[Patent document 3] Japanese Laid-Open Patent Publication No.2005-110201
[Patent document 4] Japanese Laid-Open Patent Publication No.2002-271303

### [Brief Summary of the Invention]

### [Problems to be solved by the invention]

In the wireless communication system such as the BAN described above, shadowing greatly affects the wireless communications. For example, in the BAN system, respective nodes performing the wireless communications are disposed on a living body or in the vicinity of the living body (e.g., distanced several meters from the living body). With this arrangement, in the BAN system, a posture of the living body attached with sensor devices changes, and hence a part such as an arm and a foot of the living body intercepts the transmission path of each communication node, i.e., the sensor device etc, with the result that the shadowing occurs as the case may be.

The shadowing tends to occur and continue non-statistically as compared with fading. Namely, the shadowing is more difficult to predict its occurrence than the fading. Further, the shadowing has a possibility of causing a communication-disabled status for a much longer period of time than the fading.

In the conventional wireless communication system, the transmission status could not be precisely grasped during the occurrence of the shadowing such as this. If unable to precisely grasp the transmission status, such a possibility might arise that a device like a gateway device which collects pieces of biometric information from other sensor devices is unable to properly collect the biometric information acquired by other sensor devices.

It is an object of the present disclosure to provide, in view of these problems, a technology of accurately grasping the transmission status even when the shadowing occurs.

### [Means for solving the Problems]

According to a mode of the present disclosure, the following configuration is adopted in order to solve the problems described above.

According to a first mode of the present disclosure, a wireless communication device includes transmitting means wirelessly transmitting test signals a predetermined number of times at an interval shorter than a transmission interval of data signals and measuring means measuring reception status of a plurality of acknowledgement signals transmitted back in response to the respective test signals transmitted by the transmitting means.

Another mode of the present disclosure may be a method of realizing any one of the configurations described above, may also be a program and may further be a computer readable storage medium recorded with such a program.

### [Effects of the invention]

According to the modes described above, it is feasible to provide the technology of accurately grasping the transmission status even when the shadowing occurs.

### [Brief Description of the Drawings]

[FIG.1] A view illustrating an example of an architecture of a BAN system.
[FIG.2] A diagram illustrating a configuration of a wireless communication processing unit in a first working example.
[FIG.3] A diagram illustrating an example of a partial configuration of a gateway sensor device 15 in a second working example.
[FIG.4] A diagram illustrating an example of a frame structure of a test signal.
[FIG.5] A sequence chart illustrating an example of a communication sequence in the second working example.
[FIG.6] A flowchart illustrating an operational example of a gateway sensor device 15 in the second working example.
[FIG.7] A flowchart illustrating an operational example of a sensor device 10 in the second working example.
[FIG.8] A diagram illustrating an example of a partial configuration of the gateway sensor device 15 in a third working example.
[FIG.9] A diagram illustrating an example of a frame structure of a changeover request signal.
[FIG.10A] A flowchart illustrating an operational example of the gateway sensor device 15 in the third working example.
[FIG.10B] A flowchart illustrating an operational example of the gateway sensor device 15 in the third working example.
[FIG.11] A diagram illustrating an example of a partial configuration of the gateway sensor device 15 in a modified example.
[FIG.12] A sequence chart illustrating a communication sequence in the modified example.

### [Mode for Carrying out the Invention]

A wireless communication device in an embodiment will hereinafter be described with reference to the drawings in a way that gives specific examples. The following working examples will exemplify the wireless communication device applied to a BAN system, however, the wireless communication device in the embodiment does not limit a system etc to be utilized. Further, the wireless communication device in the working examples given below may or may not have a sensing function. It should be noted that configurations in the respective working examples, which will hereinafter be described, are exemplifications, and the embodiment is not limited to the configurations in the following working examples.

### [First Working Example]

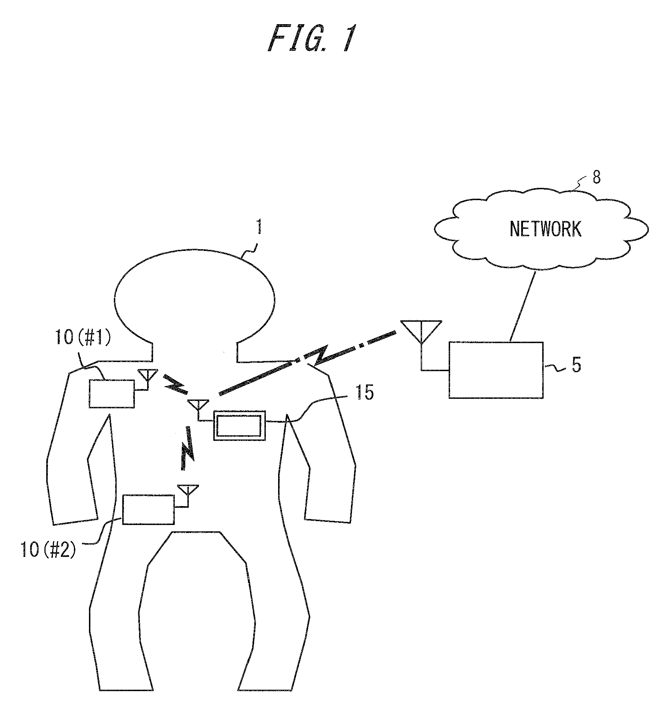

A wireless communication device in the first working example will hereinafter be described. The wireless communication device in the first working example is applied to the BAN system as depicted in FIG. 1. FIG. 1 is a view illustrating an example of an architecture of the BAN system. The BAN system includes sensor devices 10 (#1 and #2), a gateway sensor device 15, a gateway device 5, etc. The sensor devices 10 and the gateway sensor device 15 are attached to a living body 1 and thus measure, e.g., biometric information. Note that the gateway sensor device 15 may not have the sensing function.

The sensor devices 10 (#1 and #2) perform wireless communications with the gateway sensor device 15 via an air space serving as a transmission path. The sensor devices 10 (#1 and #2) transmit the measured biometric information to the gateway sensor device 15 through the wireless communications.

The gateway sensor device 15 collects pieces of biometric information from the sensor devices 10 (#1 and #2). The gateway sensor device 15 performs wireless communications with the gateway device 5 via the air space serving as the transmission path, thereby transmitting the collected pieces of biometric information to the gateway device 5.

The gateway device 5 is, e.g., installed in a position spaced away from the living body 1 in a wireless-communication-enabled range with the gateway sensor device 15. The gateway device 5 transmits the pieces of biometric information transmitted from the gateway sensor device 15 to a network 8 etc. It should be noted that the embodiment does not limit the information (biometric information etc) dealt with in the BAN system.

The wireless communication device in the first working example corresponds to a wireless communication processing unit provided in each of the sensor devices 10, the gateway sensor device 15 and the gateway device 5, which are included in the BAN system. The wireless communication processing unit serving as the wireless communication device in the first working example will hereinafter be described.

### [Wireless Communication Processing Unit]

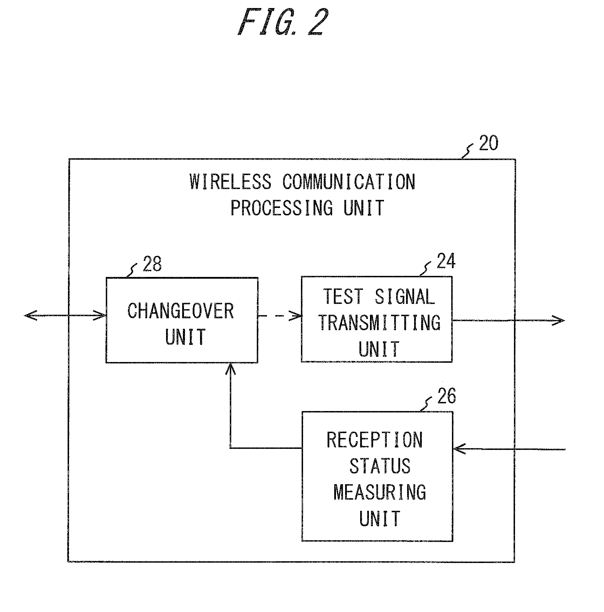

FIG. 2 is a diagram illustrating a configuration of the wireless communication processing unit in the first working example.

A wireless communication processing unit 20 in the first working example generates high-frequency signals for carrying transmission information and outputs the generated high-frequency signals to an antenna element. On the other hand, the wireless communication processing unit 20 processes the signals received by the antenna element, then extracts reception information from within these received signals and transmits the extracted reception information to other unillustrated processing units. The wireless communication processing unit 20 includes a test signal transmitting unit 24 and a reception status measuring unit 26.

The test signal transmitting unit 24 executes a test signal transmitting process in response to an instruction given from another processing unit. The test signal transmitting unit 24 converts, in this test signal transmitting process, test data into a test signal by coding and modulating the test data, and so on. The test data contains, e.g., information which specifies the test data, address information of a communication partner device, etc. The test signal transmitting unit 24 transmits the high-frequency signals to an antenna element so that the test signals are wirelessly transmitted a predetermined number of times at a predetermined transmission interval shorter than a data signal transmission interval. Herein, the "data signal" is defined as a signal containing the transmission data such as the biometric information, and the "data signal transmission interval" is defined as a parameter utilized in the device on the side of transmitting the data signal and represents a period of time till a next data signal is transmitted since the data signal of the last time has been transmitted. The data signal transmission side device sequentially transmits the data signals at the data signal transmission interval thereof.

The reception status measuring unit 26 measures reception statuses of a plurality of acknowledgement signals transmitted back in response to the test signals that are generated and wirelessly transmitted the predetermined number of times by the test signal transmitting unit 24. Hereafter, this acknowledgement signal will be abbreviated to ACK signal or simply ACK. The communication partner device, which receives the test signals transmitted the plurality of times from the device including the wireless communication processing unit 20 in the first working example, transmits the ACK signal in response to each of the normally-received test signals. The reception status measuring unit 26, upon receiving the plurality of ACK signals in the signals received by the antenna element, measures each reception status as to whether each ACK signal is normally received or not.

Thus, in the first working example, the test signals are wirelessly transmitted the predetermined number of times at the predetermined transmission interval shorter than the transmission interval of the data signals. Namely, in the first working example, a transmission status for the predetermined period of time till the transmission of all of the test signals has been completed, is checked by the test signals. Note that the "predetermined period of time" is a length of time that is long to such an extent as to enable a grasp of the transmission status of fading, shadowing, etc. It is desirable that this predetermined period of time is set by taking into consideration a length of time which can allow, e.g., a communication-disabled status of the data signals.

In this respect, when there occurs the shadowing which involves an increased number of cases of causing a longer period of time of adversely affecting the transmission status than by the fading, there is a tendency of decreasing a count of the test signals that are normally received by the communication partner device in the test signals transmitted the predetermined number of times.

Measured in the first working example are the reception statuses of the plurality of ACK signals transmitted in response to these respective test signals. For example, in the case of the occurrence of the fading, a normal reception count of the ACK signals tends to become smaller than in the case of a preferable transmission status. In the case of the occurrence of the shadowing, the normal reception count of the ACK signals tends to become smaller than in the case of the occurrence of the fading.

Therefore, according to the first working example, the transmission status can be accurately grasped even when in the occurrence of the shadowing which involves the increased number of cases of causing the long period of time of adversely affecting the transmission status. Further, it is feasible to precisely grasp the transmission status even when in the occurrence of the fading which involves an increased number of cases where the period of time of adversely affecting the transmission status is shorter than by the shadowing.

The wireless communication processing unit 20 in the first working example may be further provided with a changeover unit 28 in addition to the configuration described above. In this case, the reception status measuring unit 26 transmits pieces of information on the measured reception statuses of the plurality of ACK signals to the changeover unit 28.

The changeover unit 28, when receiving the information on the reception statuses of the plurality of ACK signals, determines based on the reception statuses of the plurality of ACK signals whether directivity of the antenna of the self-device needs changing over or not. For example, because of the reception status being worse than a predetermined condition, in the case of presuming the occurrence of the shadowing which involves the increased number of cases of causing the long period of time of adversely affecting the transmission status, the changeover unit 28 changes over the directivity of the antenna of the gateway device.

According to the configuration such as this, the transmission status can be precisely grasped even when the shadowing occurs, and hence the self-device can be prevented from being disabled from the wireless communications with other devices by changing over the directivity of the antenna.

### [Second Working Example]

The wireless communication device in a second working example will hereinafter be described. The wireless communication device in the second working example corresponds to the gateway sensor device 15 depicted in FIG. 1. The following discussion will focus on items related to the wireless communication process with the sensor devices 10 with respect to the gateway sensor device 15 serving as the wireless communication device in the second working example.

### [Configuration of Device]

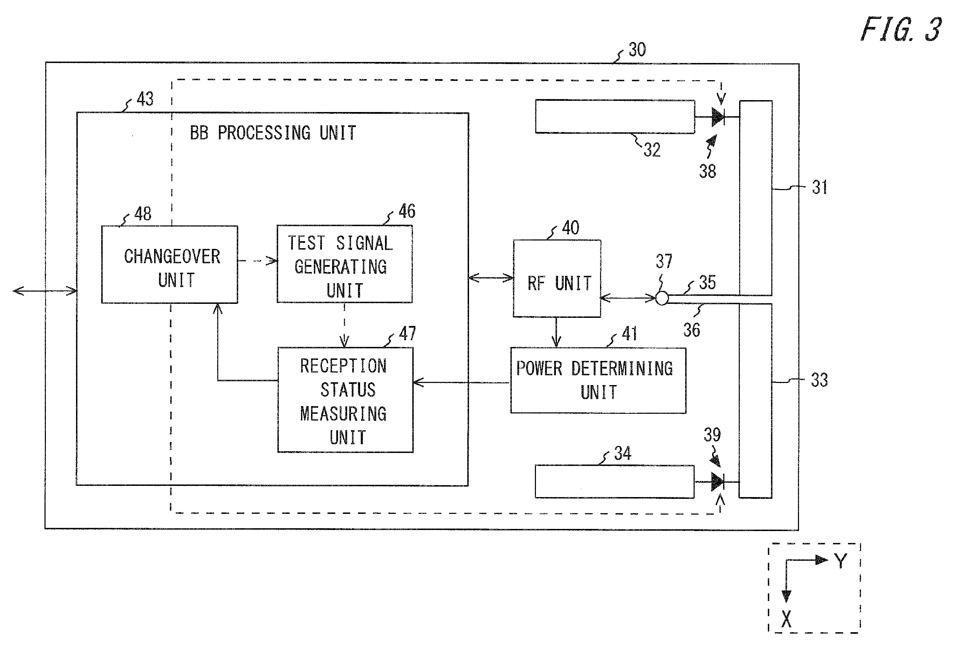

FIG. 3 is a diagram illustrating an example of a partial configuration of the gateway sensor device 15 in the second working example. The gateway sensor device 15 in the second working example includes, as depicted in FIG. 3, antenna elements 31, 32, 33, 34, a radio frequency unit (which will hereinafter be abbreviated to an RF unit) 40, a power determining unit 41, a baseband processing unit (which will hereinafter be abbreviated to a BB processing unit) 43, etc. The RF unit 40, the power determining unit 41 and the BB processing unit 43 are realized by software components or hardware components or combinations thereof, respectively (refer to Paragraph [Others]). FIG. 3 does not illustrate positions where the RF unit 40, the power determining unit 41 and the BB processing unit 43 are installed.

Electronic devices such as transistors, capacitors, resistors, IC (Integrated Circuit) and LSI (Large Scale Integration) are fixed and connected via wires on a circuit board 30. The present embodiment does not limit a structure, a shape, etc of this circuit board 30.

The antenna elements 31, 32, 33, 34 are respectively conductors and are attached to positions as illustrated in FIG. 3 on the circuit board 30. One ends of the antenna elements 31, 33 are connected to a feeding point 37 via a feeding line 35 or 36. The antenna elements 31, 33 are each supplied with electricity from the feeding point 37.

The antenna element 32 is connected to the antenna element 31 via a high-frequency switch 38. The antenna element 34 is connected to the antenna element 33 via a high-frequency switch 39. Each of the high-frequency switches 38, 39 is composed of, e.g., a PIN semiconductor diode. A changeover unit 48 of the BB processing unit 43 controls biases, whereby the high-frequency switches 38, 39 are each changed over from an ON-status to an OFF-status and vice versa. In the case of the ON-status, the high-frequency switch 38 electrically connects the antenna element 31 and the antenna element 32 to each other. In the case of the OFF-status, the high-frequency switch 38 electrically disconnects the antenna element 31 and the antenna element 32 from each other. Similarly, in the case of the ON-status, the high-frequency switch 39 electrically connects the antenna element 33 and the antenna element 34 to each other. In the case of the OFF-status, the high-frequency switch 39 electrically disconnects the antenna element 33 and the antenna element 34 from each other.

The gateway sensor device 15 in the second working example changes over the ON-status and the OFF-status of the high-frequency switches 38, 39, thereby changing over the directivity of the antenna. According to the layout of the antenna elements in the second working example, if both of the high-frequency switches 38, 39 are set in the OFF-status, the directivity having a strong sensitivity in a +Y direction (which will hereinafter be termed a directivity type 1) in FIG. 3 is realized. The high-frequency switch 38 is set in the ON-status, while the high-frequency switch 39 is set in the OFF-status, in which case, as compared with the directivity type 1, a directivity having the strong sensitivity in a -X direction (which will hereinafter be termed a directivity type 2) in FIG. 3 is realized. The high-frequency switch 38 is set in the OFF-status, while the high-frequency switch 39 is set in the ON-status, in which case, as compared with the directivity type 1, the directivity comes to have the strong sensitivity in a +X direction (which will hereinafter be termed a directivity type 3) in FIG. 3. Both of the high-frequency switches 38, 39 are set in the ON-status, in which case the directivity comes to have substantially the same sensitivity in each of ±X directions and ±Y directions (which will hereinafter be termed a directivity type 4). In the second working example, a directivity type count M of the antenna is "4".

The RF unit 40 receives a baseband signal from the BB processing unit 43 and converts this baseband signal into a high-frequency signal having a predetermined frequency. The RF unit 40, after amplifying the thus-converted high-frequency signal into a predetermined level of electric power, transmits the amplified high-frequency signal to the antenna elements 31, 33 via the feeding point 37. On the other hand, the RF unit 40, when receiving the reception signals from the antenna elements 31, 33 via the feeding point 37, amplifies the reception signals while reducing noises. The RF unit 40 converts the thus-amplified reception signals into the baseband signals, and transmits these baseband signals to the BB processing unit 43.

The power determining unit 41 determines reception power of the reception signal received from the RF unit 40. Information on this reception power involves using, e.g., reception power strength (RSSI: Received Signal Strength Indicator). The power determining unit 41 transmits the determined reception power information to the BB processing unit 43.

The BB processing unit 43 processes the transmission data transmitted from another unillustrated processing unit and the reception signals transmitted from the RF unit 40. The BB processing unit 43 includes a test signal generating unit 46, a reception status measuring unit 47, a changeover unit 48, etc.

The test signal generating unit 46 starts, in response to an instruction given from another processing unit, a test signal generating process that will be explained as below. The test signal generating unit 46 converts the test data into the baseband signals by coding and modulating the test data. The signals including the test data will hereinafter be generically termed the test signals. The test data contains, e.g., information which specifies the data as the test data, address information etc of the communication partner device and is retained beforehand. The test signal generating unit 46, on the occasion of generating the test signal, maps the test data to a predetermined location of a wireless frame. The test signal generating unit 46 generates the test signals so that the test signals including the test data are wirelessly transmitted consecutively the predetermined number of times at the predetermined transmission interval shorter than the transmission interval of the data signals. The thus-generated test signals are transmitted to the RF unit 40.

A transmission count of the test signals is set to, e.g., "100" [times] , and the transmission interval of the test signals is set to, e.g., 1 millisecond [ms]. The transmission interval and the transmission count of the test signals are previously retained. A method of determining the transmission interval and the transmission count of the test signals will be described later on.

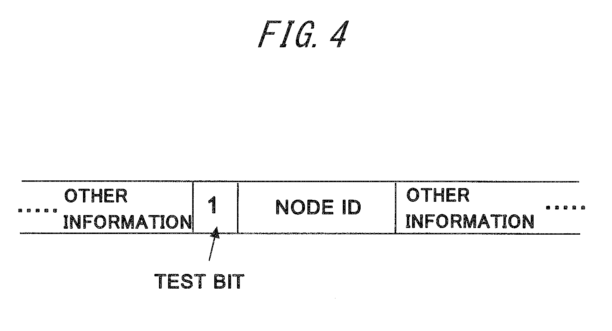

FIG. 4 is a diagram illustrating an example of a frame structure of the test signal. The test signal contains a test bit (1 bit) for distinguishing the test signal from others, a node ID, i.e. , which identifies an address of the sensor devices 10 serving as the communication partner device, and so on. It should be noted that the present embodiment does not limit the frame structure of the test signal, and it is sufficient that the frame contains the information for distinguishing between the test signal and the data signal.

The reception status measuring unit 47 measures the reception statuses of the plurality of ACK signals transmitted back in response to the test signals that are generated and wirelessly consecutively transmitted the predetermined number of times by the test signal generating unit 46 in a way that uses the reception power information of the ACK signals transmitted from the power determining unit 41. To be specific, the reception status measuring unit 47 determines whether the reception power of each ACK signal is equal to or larger than a predetermined threshold value that is previously retained, and measures a signal count of the ACK signals of which the reception power is equal to or larger than the predetermined threshold value. This predetermined threshold value involves using a value that is the same as a value used for the reception to be considered normal when receiving, e.g., the data signal. Note that the reception status measuring unit 47 may also determine that the ACK signal is normally received in a manner that takes account of not only the reception power of the ACK signal but also a decoding result etc of the BB processing unit 43.

The reception status measuring unit 47 makes the measurement targeted at the ACK signals received up to predetermined time since the first test signal has been transmitted. Hereafter, the predetermined time for determining the measurement target ACK signal will be referred to as given determination time. This given determination time is determined corresponding to the predetermined transmission interval and the predetermined transmission count for wirelessly transmitting the test signals. The given determination time may be retained beforehand and may also be determined by acquiring items of information on the transmission interval and the transmission count of the test signals and based on these items of information.

Thus, the reception status measuring unit 47 does not particularly make the determination as to the individual association between the ACK signal and the test signal but measures the signal count of the ACK signals received within the given determination time. This scheme facilitates the processing. The information for associating the test signal and the ACK signal with each other is contained in each of the signals, and the association between the test signal and the ACK signal may thus be determined.

The reception status measuring unit 47 calculates a value by dividing the measured ACK signal count by the transmission count of the test signals, and sends thus-calculated value as a test successful rate to the changeover unit 48. In other words, this test successful rate corresponds to a rate of the signal count of the ACK signals transmitted back with respect to the signal count of the wirelessly-transmitted test signals. For instance, in the case of a small signal count of the test signals that can be normally received by the sensor devices 10 in a poor environment of the transmission from the gateway sensor device 15 to the sensor devices 10, the signal count of the ACK signals transmitted from the sensor devices 10 becomes small, and hence the test successful rate decreases. Further, also in such a case that the gateway sensor device 15 is unable to normally receive the ACK signals due to the poor environment of the transmission from the sensor devices 10 to the gateway sensor device 15, similarly the test successful rate decreases.

The changeover unit 48 determines to changeover the directivity of the antenna of the gateway sensor device 15, corresponding to the test successful rate sent from the reception status measuring unit 47. Specifically, the changeover unit 48 determines whether or not the test successful rate sent from the reception status measuring unit 47 exceeds a threshold value of the successful rate that is retained beforehand. The changeover unit 48, if the test successful rate is lower than the threshold value of the successful rate, determines to changeover the directivity type that is selected at the present. The changeover unit 48, if the test successful rate is equal to or larger than the threshold value of the successful rate, i.e., if the transmission status is preferable, determines to keep the directivity type selected at the present.

The changeover unit 48, when determining to changeover the directivity type, changes over the high-frequency switches 38, 39 to a status different from the present status. The changeover unit 48 changes over the statuses of the high-frequency switches 38, 39 by controlling the biases of the high-frequency switches 38, 39. For example, the changeover unit 48, when determining to changeover the directivity type if both of the high-frequency switches 38, 39 are in the OFF-status, sets the high-frequency switch 38 in the ON-status.

The threshold value of the successful rate is, it is desirable, set to a value from which deterioration of the transmission environment due to the shadowing can be discerned. As described above, the fading tends to be small in cycle of the power fluctuations as compared with the shadowing, and hence there is a high possibility of returning to the preferable transmission environment without changing over the directivity. Accordingly, for instance, the test successful rate is previously measured when the fading occurs, and a value larger than this test successful rate is set as the threshold value of the successful rate. It should be noted that the present embodiment does not exclude phenomena such as the fading other than the shadowing from the target phenomena but detects a phenomenon exhibiting a relatively long period of time of adversely affecting the transmission environment.

The changeover unit 48, when changing over the directivity type by changing over the statuses of the high-frequency switches 38, 39, requests the test signal generating unit 46 to execute again the test signal generating process. With this operation, based on the antenna directivity after being changed over, the test signals generated by the test signal generating unit 46 are wirelessly consecutively transmitted the predetermined number of times at the predetermined transmission interval shorter than the transmission interval of the data signals.

At this time, the changeover unit 48 sets a predetermined interval between the test signal transmitting process based on the directivity type before being changed over and the test signal transmitting process based on the directivity type after being changed over. Hereafter, this predetermined interval is referred to as a test execution interval. This test execution interval is provided for preventing the ACK signal in response to the test signal of the last time from being intermingled with the ACK signal in response to the test signal of this time. Note that each signal is provided with the information for associating the test signal and the ACK signal with each other, and the interminglement may be prevented by determining the association between the test signal and the ACK signal.

The changeover unit 48 sequentially changes over the directivity types till the test successful rate exceeds the threshold value of the successful rate. Hereafter, the processes of transmitting the test signal, changing over the directivity and repeating the transmission and the changeover will be termed a test signal transmission mode, and other processes will be termed a normal processing mode as the case may be.

### [Transmission Interval and Transmission Count of Test Signal, and Test Execution Interval]

The transmission interval and the transmission count of the test signals and the test execution interval, which are utilized in the test signal generating unit 46, are determined in, e.g., the following manner.

It is desirable that the test signal transmission mode is terminated in a short period of time. This is because the deterioration of the transmission status is promptly detected to changeover the directivity, whereby the gateway sensor device 15 collects pieces of biometric information requiring urgency from the sensor devices 10 as soon as possible. Further, it is because a compression of the wireless resources due to the test signals is restrained down to the minimum to the greatest possible degree.

On the other hand, the changeover unit 48 iterates the test signal transmission mode till selecting the directivity type in which the test successful rate becomes equal to or larger than the threshold value of the successful rate. Namely, the longest continuation time of the test signal transmission mode is defined as a period of time expended for the execution with respect to all of the directivity types realizable by the sensor device 1.

Accordingly, it is desirable that the transmission interval and the transmission count of the test signals and the test execution interval are determined in a way that takes account of at least a type count M of the directivity types realizable by the sensor device 1 and a period of time for which to allow the communication-disabled status of the data signal. With this determination, the transmission interval of the test signals is set shorter than the transmission interval of the data signals. Moreover, to give another point of view, the transmission count of the test signals corresponds to the time for determining the transmission environment and is therefore set to a count enabling the detection of the phenomenon such as the shadowing that exhibits the relatively long period of time of adversely affecting the transmission environment. Moreover, the test execution interval is set to a period of time enabling the prevention of intermingling the ACK signal in response to the test signal of the last time with the ACK signal in response to the test signal of this time.

Exemplified herein is a case in which the time making allowable the communication-disabled status of the data signals is set to 1 second [s]. The gateway sensor device 15 in the second working example is capable of realizing four directivity types. Hence, for instance, the transmission interval of the test signals is set to 1[ms], the transmission count of the test signals is set to 100 [times], and the test execution interval is set to 100[ms]. In this case, the longest continuation time of the transmission of the test signals is given by 800 [ms] (=(1[ms] x 100 [times] + 100 [ms]) × 4), which can sufficiently satisfy the requirement of 1[s] described above.

### [Operational Example]

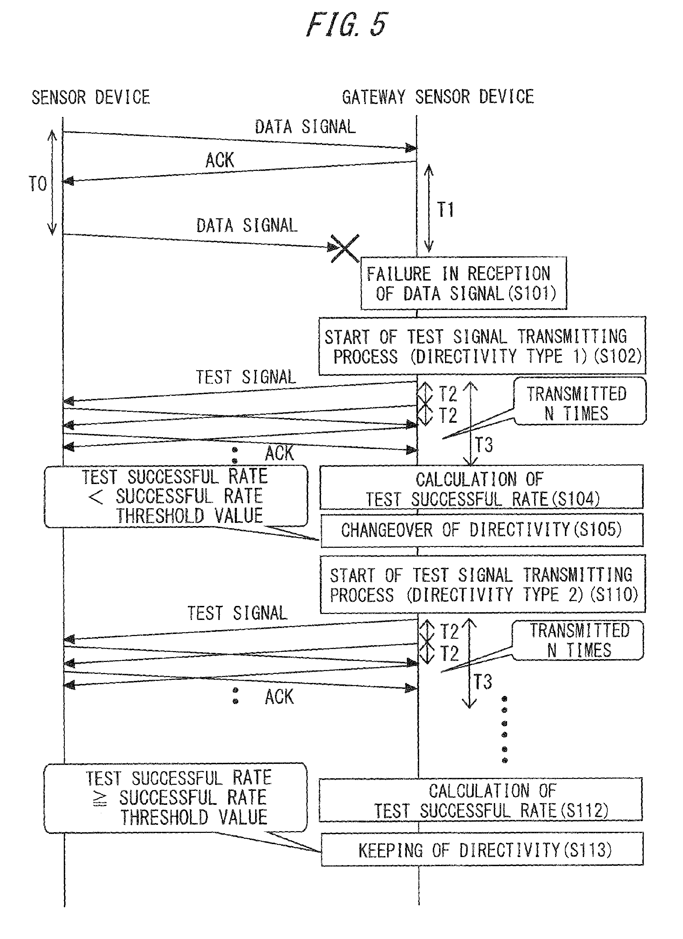

An operational example of the gateway sensor device 15 in the second working example will hereinafter be explained. FIG. 5 is a sequence chart illustrating an example of a communication sequence in the second working example.

Each of the sensor devices 10 (#1 and #2), when reaching the transmission timing of the self-device, wirelessly transmits the data signals containing the biometric information etc to the gateway sensor device 15. In the case of the preferable transmission status, the gateway sensor device 15 can normally receive the data signals in the status of having the larger reception power than the predetermined threshold value. The gateway sensor device 15, upon normally receiving the data signals, wirelessly transmits the ACK signals to the sensor devices 10.

Each of the sensor devices 10 (#1 and #2), after an elapse of a predetermined interval (T0) since when transmitting the data signals last time, transmits the next data signal to the gateway sensor device 15. At this time, an assumption is that this data signal does not normally reach the gateway sensor device 15 for some reason such as the deterioration of the transmission environment.

The gateway sensor device 15, if unable to receive the data signal having the larger power than the predetermined threshold value even after an elapse of predetermined time (T1) since the ACK signal has been transmitted last time, determines that the reception of the data signal proves to be a failure (S101). This reception timing is determined depending on whether or not the predetermined time T1 elapses since the data signal has been received last time from the sensor device 10.

The gateway sensor device 15, when determining that the data signal is not normally received, generates the test signal containing the test bit, the address information etc of the sensor device 10 as the destination. The gateway sensor device 15 wirelessly consecutively transmits the test signals the predetermined number of times (N times) at the predetermined transmission interval (T2) shorter than the above transmission interval (T0) of the data signals (S102). The test signal is transmitted based on the radiation directivity of the directivity type selected at that time. For example, if both of the high-frequency switches 38, 39 are in the OFF-status, this test signal is transmitted based on the radiation directivity of the directivity type 1.

Each of the sensor devices 10, upon receiving the test signals which are transmitted a plural number of times consecutively, immediately transmits back the ACK signal in response to each test signal that is addressed to the self-device and is normally received. All of the test signals can be normally received, in which case the same number, i.e., N-pieces of ACK signals as the number of the test signals are transmitted back.

At this time, the antenna of the gateway sensor device 15 is set to the directivity type 1. The gateway sensor device 15 receives the plurality of ACK signals with the directivity of the directivity type 1, and determines the reception power etc of each ACK signal. The gateway sensor device 15 determines whether or not the reception power of each ACK signal exceeds the predetermined threshold value that is retained beforehand in the way of being targeted at the ACK signals received within the given determination time (T3) since the transmission of the test signal has been started. The gateway sensor device 15 counts the number of the ACK signals of which the reception power exceeds the predetermined threshold value as the signal count of the normally-received ACK signals. The gateway sensor device 15 calculates a test successful rate (P) by dividing the thus-counted ACK signal count by a transmission count (N) of the test signals (S104). In other words, the gateway sensor device 15 calculates a ratio of the normal reception count of the transmitted-back ACK signals to the transmission count of the test signals.

The gateway sensor device 15 compares this test successful rate (P) with a pre-retained successful rate threshold value (Pth). The gateway sensor device 15, when determining that the test successful rate is smaller than the successful rate threshold value, changes over the directivity of the antenna (S105). For example, the directivity is changed over to the directivity type 2 from the directivity type 1 remaining selected so far.

The gateway sensor device 15 executes again the transmission of the test signals with the radiation directivity of the directivity type 2 after being changed over. As a result, with the directivity type 2, the test signals are wirelessly consecutively transmitted the predetermined number of times (N times) at the predetermined transmission interval (T2) shorter than the transmission interval (T0) of the data signals (S110).

Each of the sensor devices 10, when receiving the test signals which are thus transmitted consecutively the plural number of times, in the same way as done last time, promptly transmits back the ACK signal in response to each test signal that is addressed to the self-device and normally received.

The gateway sensor device 15 receives the ACK signal transmitted from the sensor device 10 with the directivity of the directivity type 2 after being changed over. The gateway sensor device 15 calculates the test successful rate (P) with respect to the received ACK signals and, if this test successful rate (P) is smaller than the successful rate threshold value (Pth), changes over the directivity. With this operation, the directivity of the antenna is changed over to the directivity type 3 from the directivity type 2 of the last time.

The gateway sensor device 15 repeats the processes of transmitting the test signals and changing over the directivity of the antenna till the test successful rate becomes equal to or larger than the successful rate threshold value. The second working example has the configuration enabling the four directivity types to be realized, and hence the test signal is transmitted four times at the maximum.

Therefore, according to the gateway sensor device 15 in the second working example, even when there occurs a situation such as the shadowing which deteriorates the transmission status for the relatively long period of time, the data signals can be received in a way that avoids the deteriorated transmission environment by changing over the directivity. Hence, according to the second working example, it is feasible to avoid the wireless-communication-disabled status between the sensor device 10 and the gateway sensor device 15.

Note that if the test successful rate of the test signal transmitted for the first time is equal to or larger than the successful rate threshold value, the directivity remaining selected is kept in an as-is status without changing over the directivity. The case of having no necessity for changing over the directivity includes a case in which the transmission status is deteriorated only in a relatively short period of time. For instance, this case is applied to a case where the data signal is unable to be normally received due to the influence of the fading that exhibits a short cycle of the power fluctuations.

As described above, according to the second working example, the communication-disabled status is avoided by distinguishing the phenomenon exhibiting the long period of time of adversely affecting the transmission status and the phenomenon exhibiting the short period of time thereof and changing over the directivity when the time-elongate phenomenon occurs.

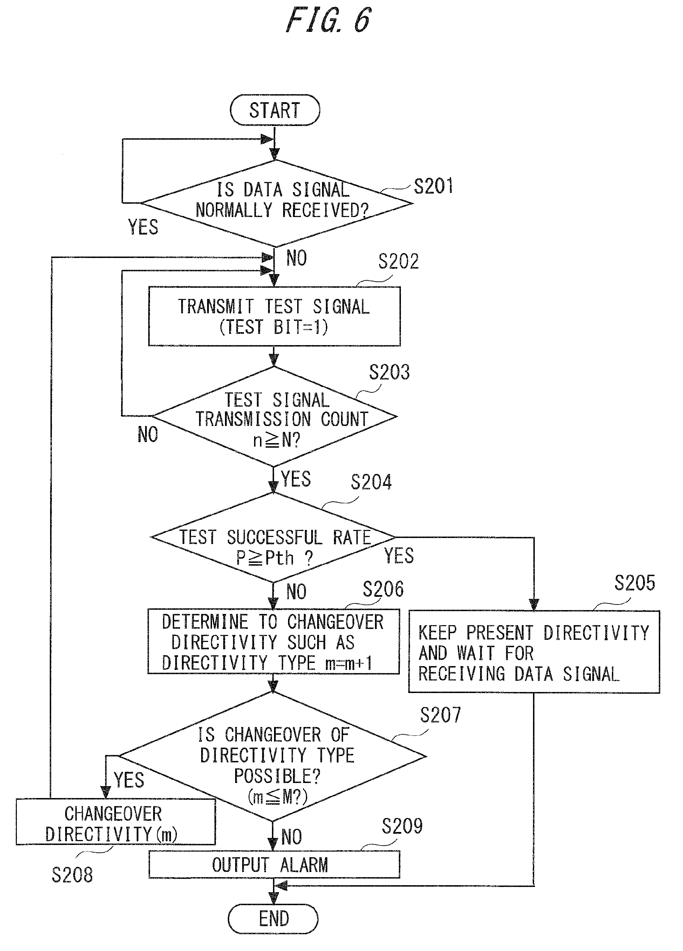

FIG. 6 is a flowchart illustrating an operational example of the gateway sensor device 15 in the second working example.

In the gateway sensor device 15, the BB processing unit 43 determines, based on the results of modulating and decoding the baseband signal transmitted from the RF unit 40 as well as on the reception power information sent from the power determining unit 41, whether the reception signal is normally received or not (S201). The BB processing unit 43, if the reception power of the data signal is lower than the predetermined threshold value, determines that the reception of the data signal proves to be the failure (S201; NO). In the gateway sensor device 15, when determining that the reception of the data signal proves to be the failure (S201; NO), the BB processing unit 43 requests the test signal generating unit 46 to execute the test signal generating process.

The test signal generating unit 46 receiving this request generates the test signal containing the test bit (1) and the address information etc of the sensor device 10 serving as the destination, and transmits this test signal to the RF unit 40. This test signal is, after being converted into the high-frequency signal and amplified by the RF unit 40, transmitted to the antenna element via the feeding point 37. At this time, both of the high-frequency switches 38, 39 are in the OFF-status. Consequently, the test signals are wirelessly consecutively transmitted the predetermined number of times (N times) at the predetermined transmission interval (T2) shorter than the transmission interval (T0) of the data signals (S202, S203).

At this time, the directivity of the antenna is set to the directivity type 1, and hence the ACK signal sent back in response to the test signal is received by the antenna elements 31 and 33 each having the directivity type 1. The ACK signal received by the antenna elements is processed by the RF unit 40 and transmitted to the BB processing unit 40. Further, the power determining unit 41 measures the reception power of the ACK signal, and the information indicating the measured reception power of the ACK signal is transmitted to the reception status measuring unit 47. At this time, the reception status measuring unit 47 is notified of a purport that the test signal generating unit 46 has started the test signal generating process.

The reception status measuring unit 47 determines whether or not the reception power of each ACK signal exceeds the predetermined threshold value that is retained beforehand in the way of being targeted at the ACK signals received within the given determination time (T3) since this notification has been received. The reception status measuring unit 47 counts the number of the ACK signals of which the reception power exceeds the predetermined threshold value as the signal count of the normally-received ACK signals. The reception status measuring unit 47 calculates the test successful rate (P) by dividing the ACK signal count that is thus counted within the given determination time (T3) since the test signal has been transmitted by the transmission count (N) of the test signals. The reception status measuring unit 47 sends the thus-calculated test successful rate (P) to the changeover unit 48.

The changeover unit 48, when receiving the test successful rate (P) from the reception status measuring unit 47, compares this test successful rate (P) with the pre-retained successful rate threshold value (Pth) (S204). The changeover unit 48, if the test successful rate is equal to or larger than the successful rate threshold value (S204; YES), determines to keep the directivity type selected at the present (S205). With this operation, the gateway sensor device 15 comes to wait for receiving the data signal from the sensor device 10.

The changeover unit 48, if the test successful rate is lower than the successful rate threshold value (S204; NO) , determines to changeover the directivity of the antenna (S206). If the antenna directivity selected at the present is the directivity type 1, the changeover to the directivity type 2 is determined. The changeover unit 48 determines whether this directivity type 2 is realizable or not (S207). The gateway sensor device 15 in the second working example has the four directivity types, and therefore the directivity type 2 is determined to be realizable (S207; YES).

The changeover unit 48, when determining that the directivity type 2 is realizable (S207; YES), changes over to the directivity type 2 from the directivity type 1 (S208). To be specific, the changeover unit 48 sets the high-frequency switch 38 in the ON-status and the high-frequency switch 39 in the OFF-status by controlling the biases.

The changeover unit 48, after an elapse of the predetermined interval (the test execution interval) since the test successful rate information has been received from the reception status measuring unit 47, requests the test signal generating unit 46 to execute again the test signal generating process (looping back to S202). The changeover unit 48 prevents the ACK signal responding to the test signal of the last time from being intermingled with the ACK signal responding to the test signal of this time by providing the test execution interval.

If the test successful rate does not become equal to or larger than the successful rate threshold value even by changing over to any directivity types, finally the test signal using the directivity type 4 is transmitted. As a result, the changeover unit 48, when determining that the test successful rate is lower than the successful rate threshold value (S204; NO), determines to changeover to the directivity type 5 from the now-selected directivity type 4 (S206).

The changeover unit 48, however, since the gateway sensor device 15 in the second working example is provided with only the directivity type 4, determines that the directivity type 5 is not realizable (S207; NO). In this case, the transmission environment thereof is unable to be avoided with the antenna directivity of the gateway sensor device 15, and hence, e.g., an alarm is output (S209). This alarm output is exemplified such as lighting up an LED provided in the gateway sensor device 15 and outputting voices.

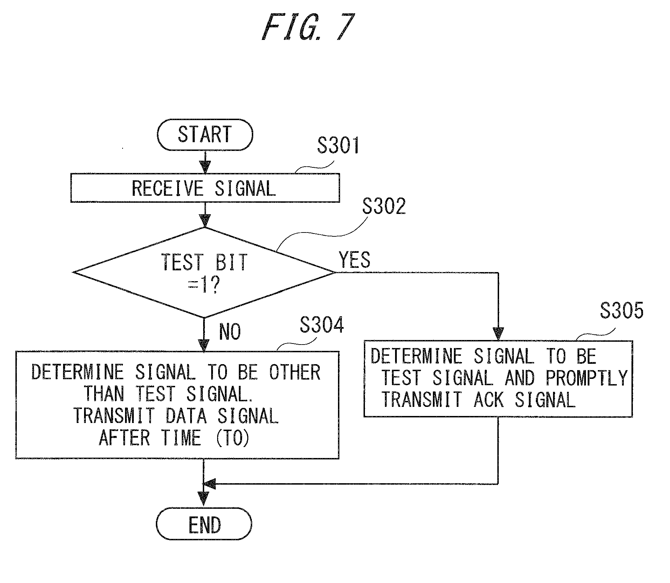

FIG. 7 is a flowchart illustrating an operational example of the sensor device 10 in the second working example. The sensor device 10, when reaching the transmission timing of the self-device, wirelessly transmits the data signal containing the biometric information etc to the gateway sensor device 15. The sensor device 10, after transmitting the data signal, comes to a standby status for the ACK signal.

The sensor device 10, upon receiving the signal addressed to the self-device (S301) , determines whether or not the test bit contained in this signal indicates the test signal (S302). The sensor device 10, when determining that this signal indicates the test signal (S302; YES), promptly transmits the ACK signal for notifying that the test signal could be normally received (S305). The sensor device 10, in the case of receiving the test signal, can know that the previously-transmitted data signal does not normally reach the gateway sensor device 15.

On the other hand, the sensor device 10, if the test bit contained in this signal proves not to indicate the test signal (S302; NO), determines that the received signal is a signal other than the test signal. The "signal other than the test signal" is exemplified such as the ACK signal responding to the data signal transmitted previously by the self-device and the data signal transmitted from the gateway sensor device 15. The sensor device 10, when determining that the signal is the ACK signal responding to the data signal transmitted previously by the self-device, transmits another data signal after the predetermined transmission interval (T0) (S304). The transmission interval (T0) of the data signal is set longer than the transmission interval (T2) of the test signal.

### [Third Working Example]

The wireless communication device in a third working example will hereinafter be described. The wireless communication device in the third working example corresponds, similarly to the second working example, to the gateway sensor device 15 illustrated in FIG. 1. The third working example is an example where the sensor device 10 serving as the destination to which the test signal is transmitted, has the function of changing over the directivity type as by the gateway sensor device 15 in the second working example. The following discussion will focus on different items from the second working example.

### [Configuration of Device]

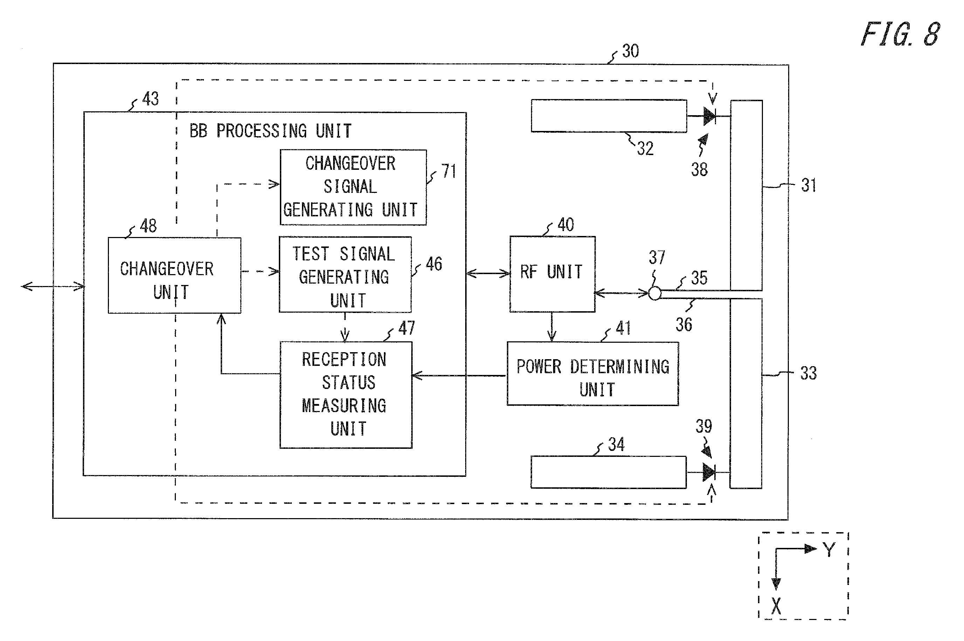

FIG. 8 is a diagram illustrating an example of a partial configuration of the gateway sensor device 15 in the third working example. The gateway sensor device 15 in the third working example further includes, as depicted in FIG. 8, a changeover signal generating unit 71 in addition to the components in the second working example. The changeover signal generating unit 71 is realized by a software component or a hardware component or a combination thereof, respectively (refer to Paragraph [Others]). This changeover signal generating unit 71 and the changeover unit 48 executing a different process from the second working example will hereinafter be explained.

The changeover signal generating unit 71 generates a changeover request signal for making a request for the changeover of the antenna directivity to the sensor device 10 becoming the communication partner device in response to an instruction given from the changeover unit 48. The changeover signal generating unit 71 transmits the generated changeover request signal to the RF unit 40.

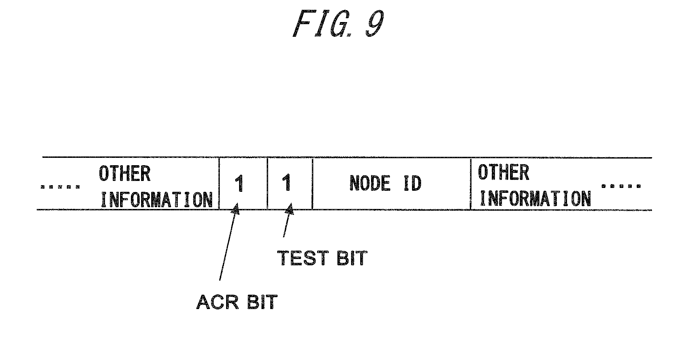

FIG. 9 is a diagram illustrating an example of a frame structure of the changeover request signal. The changeover request signal contains, in addition to the test bit (1 bit) for distinguishing the test signal from others, the node ID which indicates the address of the sensor devices 10 serving as the communication partner device, an antenna changeover request bit (which will hereinafter be abbreviated an ACR (Antenna change request) bit) for distinguishing a changeover request from others. It should be noted that the present embodiment does not limit the frame structure of the changeover request signal such as this, and it is sufficient that the frame contains the information for distinguishing the changeover request signal from others.

The changeover unit 48 in the third working example, when determining with respect to all of the directivity types that the test successful rate is lower than the successful rate threshold value, requests the changeover signal generating unit 71 to generate the changeover request signal. The changeover unit 48, when receiving the ACK signal responding to this changeover request signal within a predetermined period of time, again requests the test signal generating unit 46 for the test signal generating process in the same way as in the second working example.

### [Operational Example]

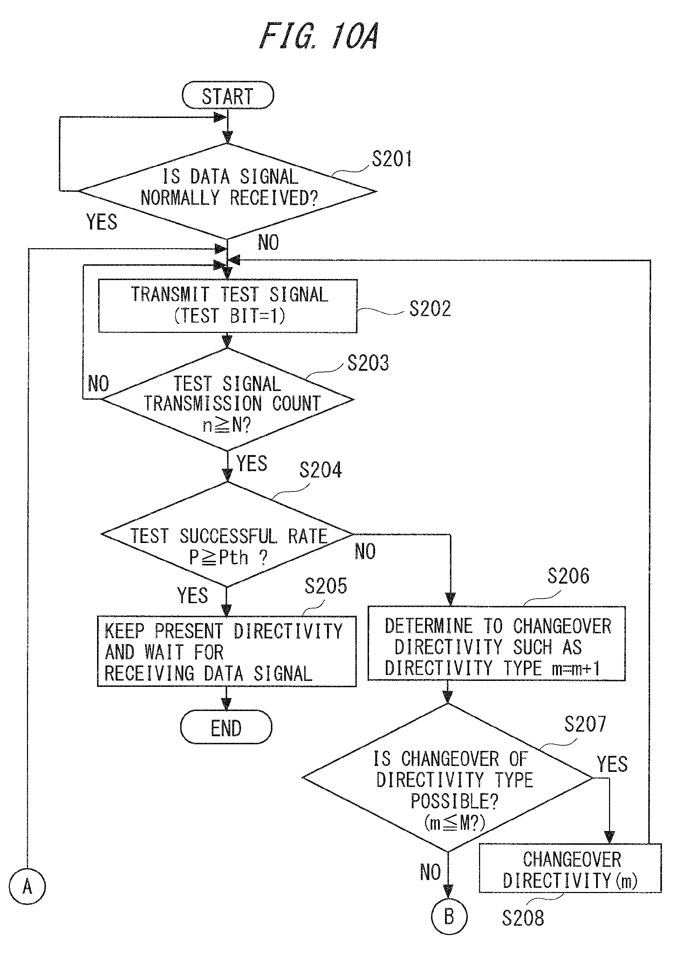

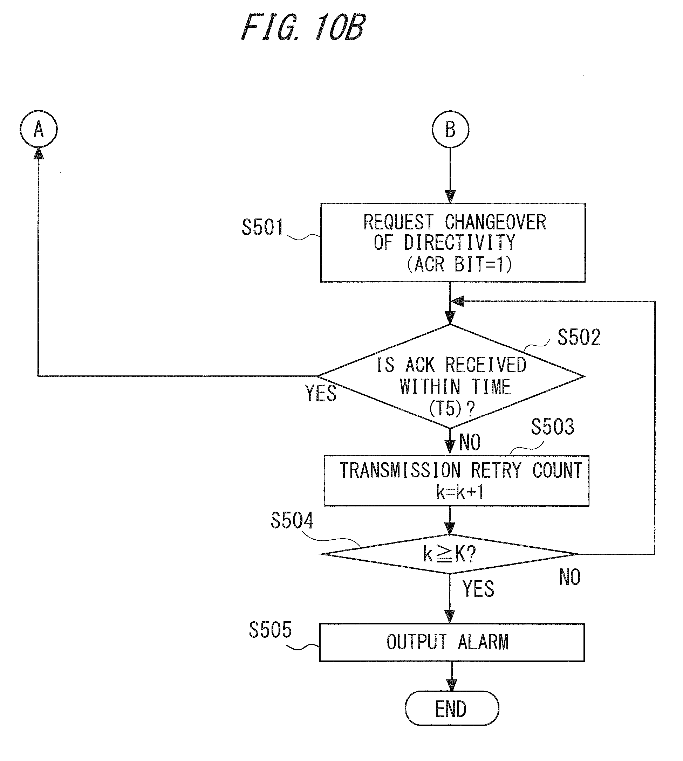

FIGS. 10A and 10B are flowcharts each illustrating an operational example of the gateway sensor device 15 in the third working example.

The operational example in the third working example is the same as the operational example in the second working example from (S201) through (S208) illustrated in FIG. 6, however, what is different from the second working example is a process after determining with respect to all of the directivity types that the test successful rate is lower than the successful rate threshold value. To be specific, the changeover unit 48, similarly to the second working example, when determining that the directivity type 5 in not realizable (S207; NO), requests the changeover signal generating unit 71 to generate the changeover request signal in the third working example.

The changeover signal generating unit 71 generates, as requested by the changeover unit 48, the changeover request signal addressed to the sensor device 10 serving as the communication partner device and transmits the generated changeover request signal to the RF unit 40. A value (1) representing the changeover request signal is set in the antenna changeover request bit of this changeover request signal. With this operation, the changeover request signal is radiated based on the directivity of the directivity type selected at that time (S501).

The sensor device 10, when receiving the changeover request signal addressed to the self-device, determines whether the antenna directivity can be changed over or not. The sensor device 10, when determining that the antenna directivity can be changed over, changes over the antenna directivity of the self-device. The sensor device 10, if successful in changing over the antenna directivity of the self-device, transmits back the ACK signal in response to the changeover request signal. Note that the sensor device 10, when determining that the antenna directivity is unable to be changed over, may transmit back nothing and may also transmit back a NACK (non-acknowledgement) signal which represents being impossible.

The changeover unit 48 comes to the standby status for the ACK signal responding to the changeover request signal for a predetermined period of time (T5) since the changeover request signal has been transmitted. The BB processing unit 43 notifies the changeover unit 48 that the ACK signal is normally received (S502; YES), the changeover unit 48 returns the antenna directivity of the self-device to the initial directivity type 1. Subsequently, the changeover unit 48 performs the control so that the transmission of the test signal and the changeover of the directivity are repeated sequentially again from the directivity type 1 (looping back to S202).

In the third working example, to begin with, in the gateway sensor device 15 defined as the node on the reception side, if it is determined with respect to all of the directivity types that the test successful rate is lower than the successful rate threshold value, the sensor device 10 defined as the node on the transmission side is requested to change over the antenna directivity. This is because the preferable transmission environment could not detected by changing over the directivity of the node on the reception side.

As a result, the sensor device 10, if provided with the function of changing over the antenna directivity, changes over the directivity. Thus, in the status where the directivity of the transmission-sided node is changed over, in the gateway sensor device 15 as the reception-sided node, the determination of the test successful rate with respect to all of the directivity types is tried again.

Therefore, according to the third working example, in a case where both of the nodes on the transmission side and the reception side of the data signals can change over the antenna directivities, it is feasible to determine a greater number of statuses of the transmission environment than in the second working example. Hence, according to the third working example, such a possibility further rises as to enable the wireless-communication-disabled status to be avoided.

While on the other hand, if the ACK signal is not normally received within the predetermined period of time (T5) from the BB processing unit 43 (S502; NO), there is made a request for retransmitting the changeover request signal to the changeover signal generating unit 71 (S503). The changeover unit 48 repeats the transmission of the changeover request signal a predetermined number of times (K) till the ACK signal responding to the changeover request signal is normally received (S504).

The changeover unit 48, if unable to receive the ACK signal even by transmitting the changeover request signal the predetermined number of times (K) (S504; YES), outputs, e.g., an alarm (S505). The "case of being unable to receive the ACK signal responding to the changeover request signal" includes a case in which the sensor device 10 is unable to normally receive the changeover request signal due to the poor transmission environment, a case in which the sensor device 10 is unable to change over the antenna directivity, and so on. This alarm output is exemplified by lighting up the LED provided in the gateway sensor device 15 and outputting the voices.

### [Modified Example]

### [Modified Example of Configuration of Antenna]

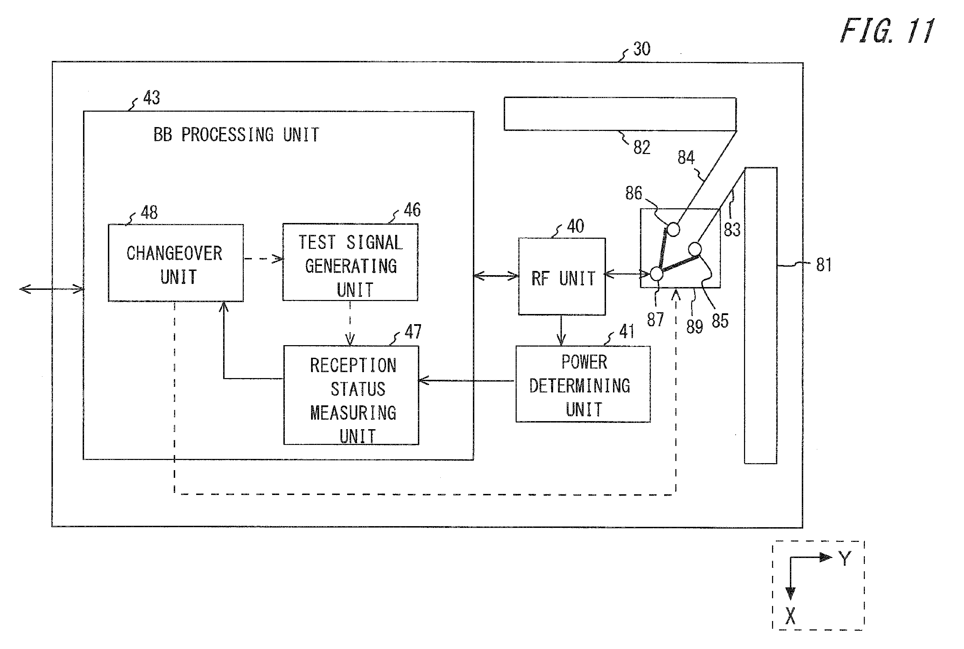

The wireless communication device in the present embodiment does not limit the antenna configuration for realizing the plurality of directivities to the antenna configurations as illustrated in the second and third working examples. For example, another available configuration is that the directivity is changed over by a switch as depicted in FIG. 11. FIG. 11 is a diagram illustrating an example of a partial configuration of the gateway sensor device 15 by way of a modified example.

Antenna elements 81, 82 are fitted to positions on the circuit board 30 as illustrated in FIG. 11. One end of the antenna element 81 is connected to a feeding point 85 via a feeding line 83. One end of the antenna element 82 is connected to a feeding point 86 via a feeding line 84. A switch 89 changes over connections of a feeding point 87 to establish the connection with the RF unit 40 with the feeding points 85, 86 in accordance with the instruction signal given from the changeover unit 48.

With this configuration, when the switch 89 connects the feeding point 87 to only the feeding point 85, the antenna directivity based on only the antenna element 81 is realized. When the switch 89 connects the feeding point 87 to only the feeding point 86, the antenna directivity based on only the antenna element 82 is realized. When the switch 89 connects the feeding point 87 to the feeding points 85, 86, the antenna directivities based on the antenna elements 81, 82 are realized. This modified example actualizes the three directivity types.

It should be noted that the present embodiment does not limit the antenna configuration to those illustrated in the second and third working examples and this modified example as well, but an available configuration is that a much larger number of antenna elements may be connected by the switch 89. Moreover, a plurality of antennas may be provided such as a linear type antenna and a flat panel antenna each taking a different shape.

Further, the second and third working examples discussed above have exemplified the gateway sensor device 15 targeted at the sensor device 10 as the communication partner device, however, the present invention may be applied to a gateway sensor device 5 targeted at the gateway sensor device 15 or the sensor device 10 as the communication partner device. In this case, not the antenna provided on the circuit board but an antenna taking the form of projecting in the air space may be provided, and there may be taken the same configurations other than the antenna as those in the second and third working examples.

### [Modified Example of Measurement of Reception Status]

The changeover unit 48 in the second and third working examples determines whether the transmission status is preferable or not by use of the value (the test successful rate) obtained in a way that divides the signal count of the ACK signals of which the reception power is equal to or larger than the predetermined threshold value by the transmission count of the test signals. The changeover unit 48 may, by way of a modified example, determine whether the transmission status is preferable or not by using an average value of the reception power of the ACK signals received for the given determination time. In this case, the reception status measuring unit 47 may calculate the average value of the reception power by employing the reception power information of the ACK signals transmitted from the power determining unit 41. Then, the changeover unit 48, if the average value of the reception power of the ACK signals is lower than the predetermined threshold value, determines to change over the directivity.

Further, two pieces of predetermined threshold values are provided, in which the changeover of the directivity may be determined if the average value of the reception power of the ACK signals is lower than the smaller threshold value, and the modulation method may be changed to increase a data rate if higher than the larger threshold value.

### [General Description]

The second and third working examples have exemplified the instance in which the gateway sensor device 15 on the reception side of the data signals takes the initiative in transmitting the test signals and changing over the directivity, however, the device on the transmission side of the data signals may take the initiative in performing these operations.

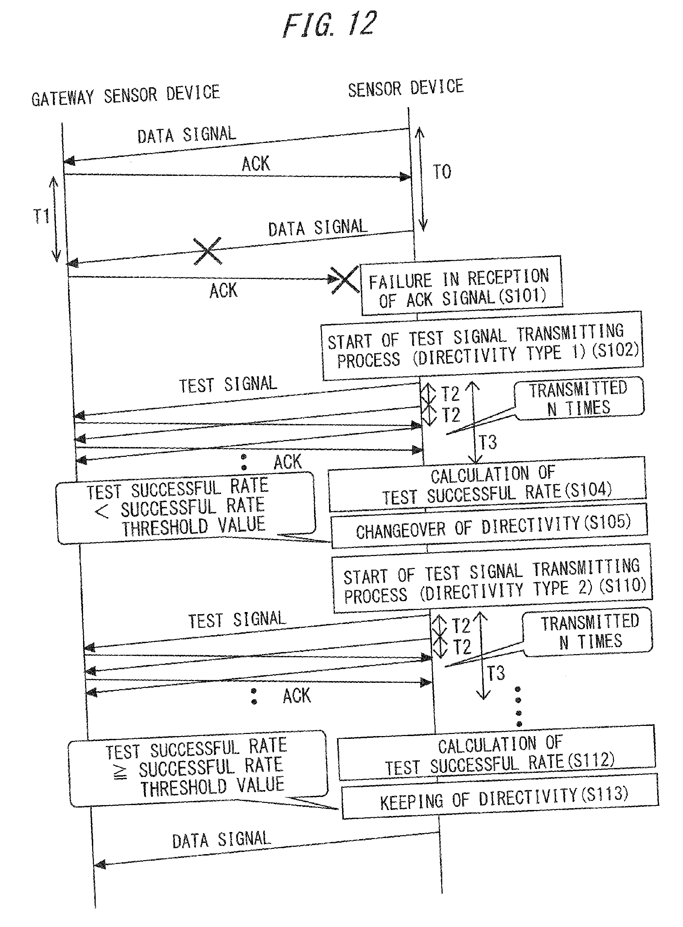

FIG. 12 is a sequence chart illustrating a communication sequence in the modified example. In the modified example of FIG. 12, the sensor device 10 on the transmission side of the data signals takes the initiative in transmitting the test signals and changing over the directivity. Namely, the sensor device 10, after wirelessly transmitting the data signal, if unable to normally receive the ACK signal responding to the data signal, stops the process of transmitting the data signal but starts the process of transmitting the test signal. The sensor device 10, if the test successful rate becomes equal to or larger than the successful rate threshold value due to the changeover of the directivity, resumes the transmission of the data signals with the directivity selected at that time.

Moreover, in the example described above, the test signal transmitting process is started if the data signal or the ACK signal is not normally received with the reception power equal to or larger than the predetermined threshold value. In the wireless communication device having the directivity changeover function, however, the test signal transmitting process may also be started when starting up the device such as starting up the power source. This contrivance enables the wireless-communication-disabled status to be avoided even if the shadowing already occurs when starting up the device.

Further, when not performing the wireless communications, the antenna directivity may also be changed over successively on a time basis. Still further, the antenna directivity may also be changed over at an arbitrary cycle by use of random numbers etc. Thus, a loss probability of the first data signal can be reduced.

### [Others]

### <Concerning Hardware Components and Software Components>

Hardware components are defined as hardware circuits which are exemplified such as a field programmable gate array (FPGA)] , an application specific integrated circuit (ASIC), a gate array, a combination of logic gates, a signal processing circuit, an analog circuit and other types of circuits.

Software components are defined as component segments (fragments) for realizing the processes described above by way of the software but do not come under any concepts of limiting languages, development environments for realizing the software. The software components are exemplified such as a task, a process, a thread, a driver, firmware, a database, a table, a function, a procedure, a subroutine, a predetermined portion of program code, a data structure, an array, a variable, a parameter etc. These software components are realized on a single or a plurality of memories (a single or a plurality of processors (e.g., a CPU (Central Processing Unit), a DSP (Digital Signal Processor), etc).

It is noted that each embodiment discussed above does not restrict the technique of realizing the respective processing units. It may be sufficient that the processing units are configured as the hardware components or the software components or the combinations thereof by the technique realizable by the normal engineers in the present technical field.

### [Description of the Numerals]

- 1: living body
- 5: gateway device
- 10: sensor device
- 15: gateway sensor device
- 20: wireless communication processing unit
- 24, 46: test signal transmitting unit
- 26, 47: reception status measuring unit
- 28, 48: changeover unit
- 31, 32, 33, 34, 81, 82: antenna element
- 37, 85, 86, 87: feeding point
- 38, 39: high-frequency switch
- 40: radio frequency unit (RF unit)
- 41: power determining unit
- 43: baseband processing unit (BB processing unit)
- 71: changeover signal generating unit
- 89: switch

## Claims

1. A wireless communication device comprising:
transmitting means wirelessly transmitting test signals a predetermined number of times at an interval shorter than a transmission interval of data signals; and
measuring means measuring reception status of a plurality of acknowledgement signals transmitted back in response to the respective test signals transmitted by the transmitting means.

2. The wireless communication device according to claim 1, further comprising:
an antenna having a plurality of directivities; and
changeover means changing over a directivity of the antenna in a way that corresponds to the reception status of the plurality of acknowledgement signals, which are measured by the measuring means.

3. The wireless communication device according to claim 2, wherein the changeover means requests the transmitting means to transmit the test signals by use of the post-changeover directivity and sequentially changes over the directivity of the antenna till the reception status of the plurality of acknowledgement signals, which is measured by the measuring means, becomes more preferable than a predetermined reception status.

4. The wireless communication device according to claim 2 or 3, wherein the changeover means, as a result of sequentially changing over the directivity of the antenna, if the reception status of the plurality of acknowledgement signals, which is measured by the measuring means, is determined not to be more preferable than the predetermined reception status with respect to all of the directivities of the antenna, requests a wireless communication device serving as a communication partner device to change over a radiation directivity.

5. The wireless communication device according to any one of claims 1 through 4, wherein the measuring means calculates a ratio of a normal reception count of the plurality of acknowledgement signals to a transmission count of the test signal as the reception status of the plurality of acknowledgement signals.

6. The wireless communication device according to any one of claims 1 through 5, wherein the transmitting means, when a reception status of the data signal is worse than a predetermined reception status or when starting the wireless communications, starts transmitting the test signal.

7. A wireless communication method by which a wireless communication device executes:
a step of wirelessly transmitting test signals a predetermined number of times at an interval shorter than a transmission interval of data signals; and
a step of measuring reception status of a plurality of acknowledgement signals transmitted back in response to the respective transmitted test signals.

8. The wireless communication method according to claim 7, wherein the wireless communication device includes an antenna having a plurality of directivities and further executes a step of changing over a directivity of the antenna in a way that corresponds to the measured reception status of the plurality of acknowledgement signals.

9. The wireless communication method according to claim 8, wherein the wireless communication device further executes a step of repeating the wireless transmission of the test signals by use of the post-changeover antenna directivity and the changeover of the antenna directivity till the measured reception status of the plurality of acknowledgement signals becomes more preferable than a predetermined reception status.

10. The wireless communication method according to claim 8 or 9, wherein the wireless communication device further executes a step of requesting, as a result of sequentially changing over the directivity of the antenna, if the measured reception status of the plurality of acknowledgement signals is determined not to be more preferable than the predetermined reception status with respect to all of the directivities of the antenna, a wireless communication device serving as a communication partner device to change over a radiation directivity.

11. The wireless communication method according to any one of claims 7 through 10, wherein there is calculated a ratio of a normal reception count of the plurality of acknowledgement signals to a transmission count of the test signal as the reception status of the plurality of acknowledgement signals.

12. The wireless communication method according to any one of claims 7 through 11, wherein the transmission of the test signal is started when a reception status of the data signal is worse than a predetermined reception status or when starting the wireless communications.
